(19) Europäisches Patentamt
European Patent Office
Office européen des brevets

(11) Publication number: **0 142 328 B1**

# (12) EUROPEAN PATENT SPECIFICATION

(45) Date of publication of patent specification: **17.04.91**

(51) Int. Cl.⁵: **C07D 213/643**, A01N 43/40

(21) Application number: **84307690.2**

(22) Date of filing: **07.11.84**

Divisional application 88200323 filed on 22.02.88.

The file contains technical information submitted after the application was filed and not included in this specification

## (54) Fluorophenoxy compounds, herbicidal compositions and methods.

(30) Priority: **10.11.83 US 550328**

(43) Date of publication of application:
**22.05.85 Bulletin 85/21**

(45) Publication of the grant of the patent:
**17.04.91 Bulletin 91/16**

(84) Designated Contracting States:
**AT BE CH DE FR GB IT LI NL SE**

(56) References cited:
**EP-A- 0 023 785**
**EP-A- 0 024 832**
**EP-A- 0 083 556**

**CHEMICAL ABSTRACTS, vol. 94, no. 21, May 25, 1981, page 709, abstract no. 174888b, Columbus, Ohio, US; & JP - A - 80 111 467**

(73) Proprietor: **THE DOW CHEMICAL COMPANY**
**2030 Dow Center Abbott Road P.O. Box 1967**
**Midland Michigan 48640-1967(US)**

(72) Inventor: **Rogers, Richard B.**
**1837 Polk Street**
**Concord California 94521(US)**

(74) Representative: **Allard, Susan Joyce et al**
**BOULT, WADE & TENNANT, 27 Furnival Street**
**London EC4A 1PQ(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates (a) to novel fluorophenoxy compounds, (b) to herbicidal compositions of such novel compounds and (c) to methods of using such compounds for the preemergent and postemergent control of grassy weeds in non-crop areas as well as in the presence of certain valuable crops such as soybeans, cotton and wheat.

BE-A-834,495, issued February 2, 1976, as well as the published German patent application equivalent thereto, viz., No. 2,546,251, published April 29, 1976, describe 2-((4-pyridinyl-2-oxy)phenoxy)-alkanoic acids, salts and esters having halo substitution in the 3- and/or 5-ring positions in the pyridine ring. Later references, e.g. published GB-A-2,026,865 disclose such compounds having trifluoromethyl substitution on the pyridine ring and EP-B-0002800 describes the enhanced effect of the D-stereoisomers of such compounds.

EP-A-0083556 discloses certain 2-((4-pyridinyl-2-oxy)phenoxy)-alkanoic acids. The compounds which are described therein do not have any substituents on the phenyl ring.

JP-55-111467 relates to certain [4-(5-trifluoromethyl-pyridin-2-yloxy)halophenoxy]alkane-carboxylic acid derivatives. The Specification discloses certain compounds which have a chlorine or bromine atom at the 2-position on the phenyl ring, or a fluorine atom at the 3-position on the phenyl ring. There is no disclosure in this specification of compounds with a fluorine atome at the 2-position on the phenyl ring.

The present invention is directed to novel fluorophenoxy compounds having herbicidal activity and which correspond to the formula

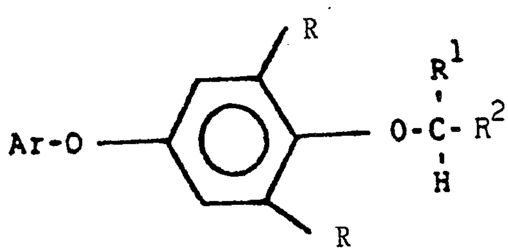

wherein Ar represents the group

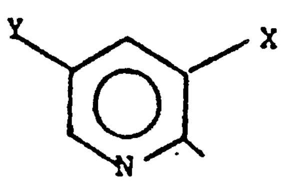

wherein X is halogen and Y is halogen, $CF_3$, $CHF_2$ or $CClF_2$;

R is H or F with the proviso that at least one K is F;

$R^1$ is a $C_1$-$C_3$ alkyl group;

$R^2$ is a carboxyl group; an agriculturally acceptable alkali metal, alkaline earth metal or ammonium salt thereof; or lower alkyl ester thereof.

The invention is also directed to the novel stereoisomers of such compounds, the R -isomers having exceptional activity.

A variety of herbicidal compounds containing substituted pyridyl and phenoxy moieties joined via a bivalent -0- or -S- are described in the art. For example, US-A-4,046,553; US-A-4,317,913; US-A-4,267,336; US-A-4,213,774; US-A-4,324,627 and US-A-4,309,547; U.S. 262,063 and 261,109, both filed July 30, 1980; EP-B-483 and EP-A-1473; EP-A-4433; EP-A-50,019; 50,097; EP-A-75,840 and EP-A- 83,556 all describe such compounds, methods of making them, compositions containing them and methods of utilizing said compositions. Given the appropriate starting materials, the compounds of this invention can be prepared by methods described in the above-mentioned prior art, and can be utilized in compositions as described in

said prior art.

The above derivatives can be made by processes generally known to those skilled in the art and as described in the above-mentioned patents.

$R^2$ is preferably a carboxylic acid group, an alkali or alkaline earth metal salt thereof, an ammonium salt thereof or a $C_{1-6}$- alkyl ester thereof, wherein $C_{1-6}$- alkyl" includes straight, branched alkyl groups containing no more than 6 carbon atoms.

In the formula for the aforementioned novel compounds, X is advantageously Cl or F, and Y is advantageously $CF_3$. The group

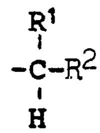

is preferably

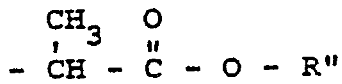

wherein R" is methyl, ethyl, propyl, isopropyl, isobutyl or n-butyl.

The compounds of the above formula, hereinafter referred to for convenience as "active ingredients", have been found to be especially active as herbicides for the control of undesired vegetation, for example, grassy or graminaceous weeds and are unexpectedly more effective than the compounds of the known art. With the compounds of this invention, it is possible to employ lower dosage rates and still obtain effective control, thus reducing plant residues and any potential environmental contamination and/or toxicological effect on fish and warm blooded animals. Accordingly, the present invention also encompasses herbicidal compositions containing one or more of these active ingredients as well as preemergent and postemergent methods of controlling undesired plant growth, especially in the presence of valuable crops. Such methods comprise applying a herbicidally-effective amount of one or more of said active ingredients to the locus of the undesired plants, that is, the seeds, foliage, rhizomes, stems and roots or other parts of the growing plants or soil in which the plants are growing or may be found.

The term "herbicide" is used herein to mean an active ingredient which controls or adversely modifies the growth of plants because of phytotoxic or other effects substantial enough to seriously retard the growth of the plant or further to damage the plant sufficiently to kill the plant.

By "growth controlling" or "herbicidally-effective" amount is meant an amount of active ingredient which causes a modifying effect and includes deviations from natural development, killing, regulation, desiccation and/or retardation.

The term "plants" is meant to include germinant seeds, emerging seedlings, rhizomes, stolons and other underground propagules, and established vegetation.

The active ingredients, i. e., new compounds, of the present invention are readily prepared by processes described in the above cited prior art and as illustrated in the following examples by choosing the appropriate starting materials. The stereoisomers are readily separated as described in EP-B-2800 referred to above.

Certain of the reactants employed to make the novel compounds of this invention are themselves novel compounds and such reactants may be made as generally described hereafter and as specifically set forth in the following examples or by methods analagous thereto, starting with known compounds.

Example 1 - Preparation of 2-(4-((3-chloro-5-(trifluoromethyl )-2-pyridinyl )-oxy)-2-fluorophenoxy)propanoic acid

A. 2-Fluoro-4-nitrophenol

To a stirred solution of 2-fluorophenol (32.3 g, 0.288 mole) in methylenechloride which was cooled to -10° C (ice-salt bath), was slowly added 90 percent nitric acid (22 g, 0.31 mole $HNO_3$) over a one-hour period. During the addition, the temperature was maintained at about -5° C. After the addition was complete, stirring was continued at 0° C for an additional hour. At the end of this period, the precipitate which had formed was filtered and washed with several portions of cold methylene chloride. G.C. and thin-layer chromatography (silica gel, 7:3 hexane-acetone) showed that this material was essentially a single compound and was the more polar and less volatile of the two products which formed in the reaction. This solid was taken up in ether, washed with water, dried ($MgSO_4$) and the solvent evaporated. The resulting solid was recrystallized from methylcyclohexane to give 13 g of a light yellow solid: m.p. = 119-121° C. NMR ($CDCl_3$) showed this to be the desired 2-fluoro-4-nitrophenol. The methylene chloride mother liquor was washed with water, dried ($MgSO_4$) and the solvent evaporated. The solid which resulted was triturated with boiling hexane (3 x 150 ml). This effectively removed all of the least polar - more volatile reaction product. This hexane solution was treated with charcoal, filtered, concentrated to about 300 ml and cooled to give 13.5 g (30 percent) of 2-fluoro-6-nitrophenol as a yellow solid: m.p. = 70-86° C.

Analysis of 2-fluoro-6-nitrophenol

| | C | H | N |
|---|---|---|---|
| Calculated: | 45.87 | 2.57 | 8.92 |
| Found: | 45.65 | 2.52 | 8.92 |

B. Methyl 2-(2-fluoro-4-nitrophenoxy)propanoic acid

A stirred mixture of 2-fluoro-4-nitrophenol (15.7 g, 0.1 mole), methyl 2-bromopropionate (16.7 g, 0.1 mole), and potassium carbonate (18.1 g, 0.13 mole) in DMSO (150 ml) was heated at 100° C (bath temperature) for 45 minutes. After cooling, the mixture was poured into ice-water (1000 ml) and the resulting mixture extracted with ether (3 x 200 ml). The ether fractions were combined, pentane (150 ml) added, and the resulting solution washed with water. The organic phase was dried ($MgSO_4$) and the solvent evaporated to give 22.5 g (92.6 percent) of the desired phenoxypropionate as a yellow liquid. This material solidified upon standing. Recrystallization from ether-hexane gave an off-white cyrstalline solid: m.p. 53-55° C; NMR ($CDCl_3$) was consistent with the assigned structure.

| Analysis: | C | H | N |
|---|---|---|---|
| Calculated: | 49.39 | 4.14 | 5.76 |
| Found: | 49.40 | 4.08 | 5.81 |

C. Methyl 2-(4-amino-2-fluorophenoxy)propionate

To a solution of the nitrophenoxypropionate prepared in B (16.6 g, 0.068 mole) in ethanol (200 ml) was added 5 percent Pd/C (1.5 g). This solution was hydrogenated on a Paar shaker (initial $H_2$ pressure = 50 psi). Hydrogen was taken up very rapidly in an exothermic reaction - the theoretical amount being consumed in less than 5 minutes. The mixture was degassed with $N_2$, filtered through celite, and the solvent evaporated to give a quantitative yield of the desired aniline as a nearly colorless oil which darkened upon standing. R.I. = 1.5189 at 25° C; NMR ($CDCl_3$); $^1H$ and $^{19}F$ were consistent with the assigned structure. This material was used directly in the next reaction.

D. The above prepared aniline (14 g, 0.066 mole) was added to a solution of concentrated HCl (25 ml) in water (75 ml) and the resulting solution cooled to about 5° C in an ice bath. To this mechanically stirred slurry was slowly dropped a solution of sodium nitrite (4.83 g, 0.07 mole) in water (10 ml). The temperature was maintained at about 7° C during the addition. By the time the addition was complete, the reaction

mixture was homogeneous. After 15 minutes of additional stirring, charcoal was added and the cold mixture filtered through celite. The filtrate was added to a 1000 ml erlenmeyer flask equipped with a mechanical stirrer and cooled in an ice bath. To this vigorously stirred solution, a solution of sodium fluoroborate (14.27 g, 0.13 mole) in water (20 ml) was added all at once. The mixture was stirred for 30 minutes, during which time a solid slowly separated. This solid was filtered, washed with several portions of ice water then dried in a vacuum oven over $P_2O_5$ for several hours at 50 °C to give 15.75 g (76.5 percent) of the diazonium tetrafluoroborate: m.p. = 122-124 °C; NMR ($CF_3CO_2H$) was consistent with the assigned structure.

E. Methyl 2-(2-fluoro-4-hydroxyphenoxy)propionate

To stirred trifluoroacetic acid (150 ml) was carefully added potassium carbonate (18.08 g, 0.13 mole). After $CO_2$ evolution ceased, the above tetrafluoroborate diazonium salt (15.75 g, 0.05 mole) was added and the resulting mixture stirred and heated at reflux for 72 hours. The reaction was followed by NMR. After cooling, excess trifluoroacetic acid was evaporated and water (350 ml) added to the residue. The resulting dark mixture was stirred at room temperature for 3 hours, then extracted with ether (3 x 200 ml). The ether phases were combined, dried ($MgSO_4$) and the solvent evaporated to give a dark viscous oil (~10 g). NMR of this material showed that the methyl ester of the propionate had been hydrolyzed. The oil was taken up in methanol (400 ml), sulfuric acid (1 g) added and the solution heated at reflux for 3 hours. Most of the methanol was evaporated and water added to the residue. This mixture was extracted with ether (3 x 150 ml), the ether extracts were combined, dried ($MgSO_4$) and the solvent evaporated. G.C. showed that 3 volatile materials were present in the ratio (peak areas) of 15:4:81 with the second peak being a shoulder off the first peak. The mixture was subjected to Kugelrohr distillation. Two fractions were collected: (1) everything up to ~110 °C (oven temperature). This consisted of essentially the first two G.C. peaks but contained a trace of peak 3. (2) Everything distilling between 110-140 °C. This consisted exclusively of peak 3 and was shown to be the desired methyl 2-(2'-fluoro-4 -hydroxyphenoxy)propionate: 5.5 9 (51 percent); NMR ($CDCl_3$) was consistent with the assigned structure; R.I. = 1.5044 at 25 °C - yellow oil.

| Analysis: | C | H |
|---|---|---|
| Calculated: | 56.07 | 5.18 |
| Found: | 54.94 | 5.16 |

F. A stirred solution of 2-fluoro-3-chloro-5-(trifluoromethyl)pyridine (2.33 g, 0.0117 mole), the above prepared phenol (2.5 g, 0.0117 mole), and potassium carbonate (2.1 g, 0.015 mole) in DMSO (25 ml) was heated at 125-140 °C (pot temperature) for 30 minutes, cooled, and poured into water (250 ml). The resulting solution was extracted with ether (3 x 100 ml). The ether phases were combined, treated with charcoal, then $MgSO_4$, filtered and the solvent evaporated to give the desired pyridyloxyphenoxypropionate as a light yellow oil (4.0 g, 87 percent): NMR ($CDCl_3$) was consistent with the assigned structure; R.I. = 1.5120 at 25 °C.

| Analysis: | C | H | N |
|---|---|---|---|
| Calculated: | 48.81 | 3.07 | 3.56 |
| Found: | 48.58 | 3.02 | 3.54 |

Example 2 - Preparation of 2(4-((3-Fluoro-5-chloro-2-pyridinyl)-oxy)-2-fluorophenoxy) propionate

A stirred mixture of 2,3-difluoro-5-chloropyridine (1.40 g, 0.0093 mole), the above phenol (1E) (2.0 g, .0093 mole) and potassium carbonate (1.39 g, 0.01 mole) in DMSO (20 ml) was heated at 140-150 °C (oil bath temperature) for 30 minutes, then cooled and poured into water (200 ml). The resulting mixture was extracted with ether (3 x 100 ml). The ether extracts were combined, and pentane (50 ml) added. This solution was washed with water (200 ml), treated with charcoal, then with $MgSO_4$, filtered and the solvent evaporated to give the desired pyridyloxyphenoxypropionate as a light yellow oil (2.6 g, 83 percent): NMR

($CDCl_3$) was consistent with the assigned structure; R.I. = 1.5349 at 25° C.

| Analysis: | C | H | N |
|---|---|---|---|
| Calculated: | 52.41 | 3.52 | 4.08 |
| Found: | 52.26 | 3.47 | 3.93 |

Example 3 - Preparation of Methyl 2-(2-fluoro-4-((3-fluoro-5-trifluoromethylpyridinyl)-2-oxy)phenoxy)-propionate

A stirred solution of methyl 2-(2-fluoro-4-hydroxyphenoxy)propionate (2.0 g, 9.34 mole), 2,3-difluoro-5-trifluoromethylpyridine (1.71 g, 9.34 mole), and potassium carbonate (2.13 g, 15.3 mmole) in dimethyl sulfoxide (40 ml) was heated at 70° C for 20 hours. After cooling, diethyl ether (100 ml) was added and the resultant mixture was stirred for five minutes. It then was filtered. The filtrate was washed sequentially with 2N HCl (2 x 100 ml) then water (3 x 100 ml). The separated ether phase was then dried ($MgSO_4$), filtered and the ether removed by distillation. A yellow oil weighing 2.8 grams (79 percent) resulted. R.I. = 1.4932 @ 25° C. NMR spectroscopy ($^1H$, $^{19}F$) confirmed the structure as that consistent with the assigned structure for the title product. The carbon, hydrogen and nitrogen content was as follows:

| Analysis: | %C | %H | %N |
|---|---|---|---|
| Calculated | 50.93 | 3.21 | 3.71 |
| Found: | 50.78 | 3.26 | 3.89 |

In a similar manner, the following derivatives were prepared:

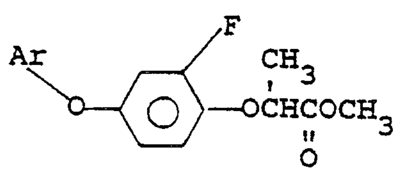

| Ar | RI or MP | Analysis Calc'd | Found |
|---|---|---|---|
| 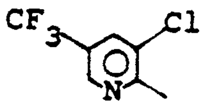 | 1.5120 | C 48.80<br>H 3.08<br>N 3.56 | C 48.58<br>H 3.02<br>N 3.54 |
| 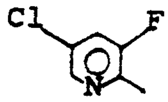 | 1.5349 | C 52.41<br>H 3.52<br>N 4.06 | C 52.26<br>H 3.47<br>N 3.93 |
| 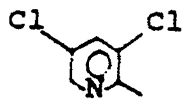 | 1.5587 | C 50.00<br>H 3.36<br>N 3.89 | C 49.76<br>H 3.17<br>N 3.85 |

Example 4 - Preparation of Methyl 2-(2,6-difluoro-4-((3-fluoro-5-(trifluoromethyl)pyridinyl)-2-oxy)phenoxy)-propionate

A. 4-Bromo-2,5-difluorophenol

A stirred mixture of 2,6-difluorophenol (20.0 g, 0.154 mole), bromine (25.57 g, 0.16 mole) and powdered iron (1 g) in methylene chloride (250 ml) was heated at reflux overnight. The cooled reaction mixture was poured into ice-water (300 ml) containing sodium bisulfite (5 g) and the organic phase separated. The aqueous phase was washed with additional methylene chloride. The organic phases were combined, dried ($MgSO_4$) and the solvent evaporated to give a light yellow oil which solidified upon standing. NMR ($CDCl_3$) of this material was consistent with the assigned structure. No additional analysis or purification was attempted. This material was used directly in the next step.

B. 4-Benzyloxy-3,5-difluorobromobenzene

A stirred mixture of 4-bromo-2,6-difluorophenol (36 g, 0.172 mole), benzylchloride (21.77 g, 0.172 mole), and potassium carbonate (25 g, 0.18 mole) in dimethylformamide (300 ml) was heated at 80-90° for four hours. After cooling, the solvent was evaporated and ether added to the residue. The insoluble inorganic salts were removed by filtration, and the solvent evaporated from the filtrate. The oily residue was subjected to bulb-to-bulb vacuum distillation on Kugelrohr apparatus to give the desired product as a nearly colorless oil (41 g, 80 percent): R.I. = 1.5602 @ 25° C; NMR ($CDCl_3$) was consistent with the assigned structure.

| Analysis: | C | H |
|---|---|---|
| Calculated | 52.20 | 3.03 |
| Found | 51.74 | 2.99 |

C. 4-Benzyloxy 3,5-difluorophenol

A stirred solution of 4-benzyloxy-3,5-difluorobromobenzene (9.87 g, 0.033 mole) in ether (100 ml) under

an atmosphere of argon was cooled to less than -70° C in a dry ice-acetone bath. To this solution was slowly added a solution of butyllithium in hexane (1.5 M, 22 ml, 0.033 moles). Stirring was continued for 30 minutes after the addition was complete. In a separate apparatus, a stirred solution of trimethylborate (3.1 g, 0.033 mole) in ether (50 ml) under an atmosphere of argon was cooled to less than -70° C. The freshly prepared benzyloxyphenyllithium formed in the first reaction solution was slowly transferred to the trimethylborate/ether solution by way of a double-tipped stainless steel needle. The addition was at such a rate that the reaction temperature was maintained at less than -66° C. After the addition was complete, the temperature was allowed to rise to room temperature. At this point, 10 percent hydrochloric acid (25 ml) was carefully added. After stirring an additional 15 minutes, the aqueous phase was separated and discarded. The organic phase was treated with 10 percent hydrogen peroxide (20 ml) and the resulting mixture stirred and heated at reflux for 24 hours. After cooling, the aqueous phase was separated and discarded. The organic layer was washed with 10 percent ferrous ammonium sulfate (25 ml), then with water (50 ml). After drying ($MgSO_4$), the ether was evaporated to yield a brown oil (7.1 g, 91 percent) which solidified upon standing. G.C. showed that this material was approximately 92 percent pure. The proton and fluorine NMR spectra were consistent with the desired structure. Recrystallization of this material from hexane/ether gave a partially purified sample: m.p. = 42-44° C. It was used without additional purification.

D. 2-(4-Benzyloxy-3,5-difluorophenoxy)-3-fluoro-5-trifluoromethylpyridine

A stirred mixture of the benzyloxyphenol (3.0 g, 0.0127 mole), 2,3-difluoro-5-trifluoromethylpyridine (2.38 g, 0.013 mole), and potassium carbonate (1.81 g, 0.013 mole) in DNF (25 ml) was heated at 90-100° for two hours. After cooling, the solvent was evaporated. Ether (100 ml) was added to the residue and the inorganic salts removed by filtration. The ether phase was treated with charcoal, then filtered through a short pad of silica gel. Evaporation of the ether gave a nearly colorless oil which solidified to a white solid upon standing. An analytical sample prepared by recrystallization from methanol/water had a m.p. = 47-48° C. Total yield = 3.6 g (71 percent).

| Analysis: | C | H | N |
|---|---|---|---|
| Calculated | 57.15 | 2.78 | 3.51 |
| Found | 56.80 | 2.72 | 3.63 |

E. 2-(3,5-Difluoro-4-hydroxyphenoxy)-3-fluoro-5-trifluoromethylpyridine

A mixture of the benzyloxyphenoxypyridine (3.4 g, 0.0085 mole) and acetic acid saturated with anhydrous HBr (50 ml) was warmed at 60-70° C for 30 minutes. The mixture was poured into water (300 ml) and the resulting mixture extracted with ether (2 x 200 ml). The combined ether extracts were washed with water (100 ml) then saturated sodium bicarbonate solution. After drying ($MgSO_4$) all the volatile material was evaporated. The resulting yellow solid was taken up in ether, treated with charcoal, then filtered through a short pad of silica gel. Removal of the solvent gave a light yellow solid (2.0 g, 76 percent). Recrystallization from methylcyclohexane gave colorless crystals: m.p. = 126-128° C. The NMR ($CDCl_3$) was consistent with the assigned structure.

| Analysis: | C | H | N |
|---|---|---|---|
| Calculated | 46.62 | 1.63 | 4.53 |
| Found | 46.88 | 1.62 | 4.63 |

F. Methyl 2-(2,6-difluoro-4-((3-fluoro-5-trifluoromethylpyridinyl)-2-oxy)phenoxy)propionate

A stirred mixture of the pyridyloxyphenol (1.7 g, 0.0055 mole), methyl 2-bromopropionate (0.92 g, 0.0055 mole), and potassium carbonate (0.83 g, 0.006 mole) in DMSO (15 ml) was heated at 70-80° for two hours, cooled, and poured into ice water (200 ml). The resulting solution was acidified with HCl, then extracted with ether (2 x 100 ml). The ether phases were combined, treated with charcoal then filtered through a short pad of silica gel. The solvent was evaporated to give the desired pyridyloxyphenoxypropionate as a light yellow oil (1.7 g, 78 percent): R.I. = 1.4828 @ 25° C; NMR ($CDCl_3$) was consistent with the assigned structure.

| Analysis: | C | H | N |
|---|---|---|---|
| Calculated | 48.62 | 2.81 | 3.54 |
| Found | 48.69 | 2.82 | 3.65 |

The compounds of the present invention have been found to be suitable for use in methods for the selective pre- and postemergent control of annual and perennial grassy weeds. These compounds, the active ingredients of the present invention, have been found to have advantage over prior art compounds in the control of annual and perennial grassy weeds in that the present compounds control such weeds at substantially lower dosage rates. In addition, the present compounds are sufficiently tolerant towards most broad leafed crops to contemplate control of grassy weeds therein at substantially commercially practicable levels, particularly so with the preferred compounds. In addition, certain of the compounds have sufficient tolerance towards cereal crops such as wheat to enable selective grassy weed control in these crops as well.

For such uses, unmodified active ingredients of the present invention can be employed. However, the present invention embraces the use of the compounds in composition form with an inert material, known in the art as an agricultural adjuvant or carrier, in solid or liquid form. Thus, for example, an active ingredient can be dispersed on a finely-divided solid and employed therein as a dust or granule. Also, the active ingredients, as liquid concentrates or solid compositions comprising one or more of the active ingredients can be dispersed in water, typically with aid of a wetting agent, and the resulting aqueous dispersion employed as a spray. In other procedures, the active ingredients can be employed as a constituent of organic liquid compositions, oil-in-water and water-in-oil emulsions or water dispersions, with or without the addition of wetting, dispersing, or emulsifying agents. suitable adjuvants of the foregoing type are well known to those skilled in the art.

The herbicidally effective concentration of the active ingredients in solid or liquid compositions generally is from 0.0003 to 95 percent by weight or more. concentrations from 0.05 to 50 percent by weight are often employed. In compositions to be employed as concentrates, the active ingredient can be present in a concentration from 5 to 98 weight percent. The active ingredient compositions can also contain other compatible additaments, for example, phytotoxicants, plant growth regulants and other biologically active compounds used in agriculture.

In further embodiments, the compounds of the present invention or compositions containing the same, can be advantageously employed in combination with one or more additional pesticidal compounds. Such additional pesticidal compounds may be insecticides, nematocides, miticides, arthropodicides, herbicides, fungicides or bactericides that are compatible with the compounds of the present invention in the medium selected for application and not antagonistic to the activity of the present compounds. Accordingly, in such embodiments, the pesticidal compound is employed as a supplemental toxicant for the same or for a different pesticidal use or as an additament. The compounds in combination can generally be present in a ratio of from 1 to 100 parts of the compound of the present invention with from 100 to 1 parts of the additional compound(s).

The active ingredients of the present invention have been found to possess desirable herbicidal activity in general against grassy weeds such as foxtail, barnyard-grass, wild oats, seedling johnsongrass and crabgrass in preemergent operations and also against the same grasses in postemergent operations while being tolerant to important broadleaf crops such as cotton, soybeans, sugarbeets and rape and in the case of certain of the compounds, certain cereal crops such as wheat. These compounds are also uniquely effective in selectively controlling perennial grassy weeds such as johnsongrass, quackgrass, bermudagrass and dallisgrass.

The active ingredients of the present invention have been found to possess particularly desirable herbicidal activity against wild oats, foxtail, barnyard-grass, crabgrass and seedling johnsongrass in

postemergent operations as well as desirable broad spectrum activity against the perennial grassy weeds listed above and at lower dosage rates than the substituted propanoates and propanols of the prior art while showing high selectivity to broadleaf crops and, in the case of certain of the compounds, wheat.

The exact rate to be applied is dependent not only on a specific active ingredient being applied, but also on a particular action desired, the plant species to be modified and the stage of growth thereof as well as the part of the plant to be contacted with the toxic active ingredient. Thus, all of the active ingredients of the present invention and compositions containing the same may not be equally effective at similar concentrations or against the same plant species.

In postemergent operations a dosage of 0.005 to 20 pounds/acre (0.0056 -22.4 kg/hectare) is generally applicable, although not all compounds are equally effective and some weeds are more difficult to control. Thus, a dosage rate in the range of 0.01 to 1.0 pound/acre (0.01-1.12 kg/hectare) is preferred in postemergent control of annual grassy weeds, while 0.05 to 5 pounds/acre (0.056-5.6 kg/hectare) is a preferred dosage range for the postemergent control of perennial grassy weeds. In applications to tolerant crops a weed controlling but less than crop damaging amount of from .005 to 1.0 lb/acre (0.0056 to 1.12 kgs/hectare) is generally employed.

In preemergent operations a dosage rate of 0.01 to 10 lbs/acre (0.011 to 11.2 kgs/hectare), preferably 0.05 to 2.0 lbs/acre (0.056 to 2.25 kgs/hectare) and most preferably 0.1 to 1 lb/acre (0.11 to 1.12 kgs/hectare) is generally employed.

The following examples illustrate effects of the compounds of this invention.

Example 5

In representative operations, each compound to be utilized in a series of tests is dissolved in acetone to one-half of the final volume (twice the final concentration) to be used and the acetone solution in each case is admixed with an equal volume of water containing 0.1 percent by weight of the non-ionic surfactant TWEEN® 20 (a polyoxyethylene sorbitan monolaurate). The compositions, generally in the nature of an emulsion, were employed to spray separate respective plant species which had been grown to a height of 2-6 inches in soil of good nutrient content in a greenhouse. Sufficient amounts were employed to provide various application rates as listed in the table. The various beds were positioned side by side and exposed to substantially identical conditions of temperature and light. Each bed was maintained so as to prevent any interaction with test compounds in different seed beds. Other portions of the plants were left untreated to serve as controls. After treatment, the plants were maintained for about two weeks under greenhouse conditions conducive for good plant growth and watered as necessary. The specific plant species, test compound and dosage and the percent postemergent control obtained are set forth in the table below. Control refers to the reduction in growth compared to the observed results of the same untreated species. Note the "NT" means "not tested".

Plant species in these tests were the following:

| Common Name | Scientific Name |
|---|---|
| Barnyardgrass (Watergrass) | *Echinochloa crusgalli* |
| Crabgrass | *Digitaria sanguinalis* |
| Yellow foxtail | *Setaria lutescens* |
| Johnson grass | *Sorghum halepense* |
| Wild Oats | *Avena fatua* |

## POSTEMERGENT CONTROL OF PLANT SPECIES

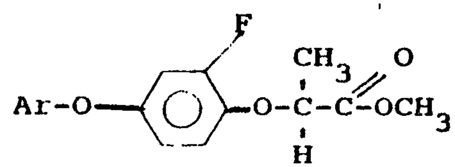

| Ar | Plant Species | Percent Control at Indicated Dosage (ppm) 250 | 125 | 62.5 | 31.25 | 15.6 | 7.8 |
|---|---|---|---|---|---|---|---|
| 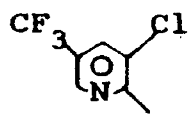 | Barnyardgrass | 100 | 100 | 100 | 100 | 65 | 10 |
| | Crabgrass | 100 | 100 | 100 | 100 | 100 | 100 |
| | Foxtail | 100 | 100 | 100 | 100 | 100 | 60 |
| | Johnsongrass | 100 | 100 | 100 | 100 | 100 | 100 |
| | Wild Oats | 100 | 100 | 100 | 100 | 20 | 0 |
| 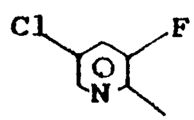 | Barnyardgrass | NT | 100 | 100 | 100 | 100 | 10 |
| | Crabgrass | NT | 100 | 100 | 100 | 100 | 100 |
| | Foxtail | NT | 100 | 100 | 100 | 100 | 95 |
| | Johnsongrass | NT | 100 | 100 | 100 | 100 | 95 |
| | Wild Oats | NT | 99 | 100 | 100 | 85 | 0 |

POSTEMERGENT CONTROL OF PLANT SPECIES (Continued)

| Ar | Plant Species | Percent Control at Indicated Dosage (ppm) 250 | 125 | 62.5 | 31.25 | 15.6 | 7.8 |
|---|---|---|---|---|---|---|---|
| 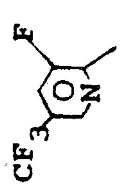 | Barnyardgrass | NT | 100 | 100 | 100 | 100 | 100 |
| | Crabgrass | NT | 100 | 100 | 100 | 100 | 100 |
| | Foxtail | NT | 100 | 100 | 100 | 100 | 100 |
| | Johnsongrass | NT | 100 | 100 | 100 | 100 | 100 |
| | Wild Oats | NT | 100 | 100 | 100 | 35 | 0 |

Example 6

So as to clearly illustrate the phytotoxic properties of the various active ingredients of the present invention applied preemergently, a controlled greenhouse experiment is described below.

The seeds of various species of plants were planted in beds of good agricultural soil in a greenhouse. A

number of compositions of the present invention, generally in the nature of an aqueous emulsion, were applied at rates listed in the table so as to deposit a predetermined amount of active ingredients uniformly throughout the surface of the bed. Another seed bed was treated only with water to serve as a control. After treatment, the seed beds were maintained for two weeks under greenhouse conditions conductive for good plant growth and watered as necessary. The specific plant species, test compound, and dosage and the percent preemergent control are set forth in the table below. Control refers to the reduction in growth compared to the observed results of the same untreated species.

PREEMERGENT CONTROL OF PLANT SPECIES

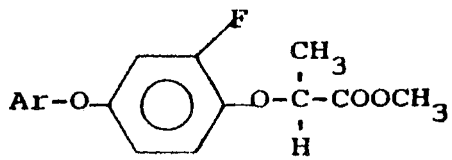

| Ar | Plant Species | Dosage (lb/acre, kg/hectare) 0.5 (0.56) | 0.25 (0.28) | 0.125 (0.14) | 0.063 (0.07) | 0.031 (0.035) |
|---|---|---|---|---|---|---|
| 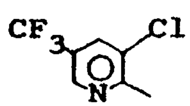 | Cotton | NT | 0 | 0 | 0 | 0 |
| | Rape | NT | 0 | 0 | 0 | 0 |
| | Soybean | NT | 0 | 0 | 0 | 0 |
| | Sugarbeet | NT | 0 | 0 | 0 | 0 |
| | Rice | NT | 100 | 100 | 75 | 10 |
| | Wheat | NT | 100 | 85 | 40 | 0 |
| | Barnyardgrass | NT | 100 | 100 | 70 | 15 |
| | Johnsongrass | NT | 100 | 100 | 100 | 95 |
| | Wild Oats | NT | 100 | 95 | 50 | 15 |
| | Yellow Foxtail | NT | 100 | 100 | 100 | 80 |

PREEMERGENT CONTROL OF PLANT SPECIES (Continued)

| Ar | Plant Species | Dosage (lb/acre, kg/hectare) 0.5 (0.56) | 0.25 (0.28) | 0.125 (0.14) | 0.063 (0.07) | 0.031 (0.035) |
|---|---|---|---|---|---|---|
| 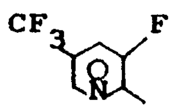 | Cotton | 0 | 0 | 0 | 0 | 0 |
| | Rape | 0 | 0 | 0 | 0 | 0 |
| | Soybean | 0 | 0 | 0 | 0 | 0 |
| | Sugarbeet | 0 | 0 | 0 | 0 | 0 |
| | Rice | 100 | 100 | 100 | 95 | 50 |
| | Wheat | 100 | 100 | 100 | 80 | 0 |
| | Barnyardgrass | 100 | 100 | 100 | 60 | 50 |
| | Johnsongrass | 100 | 100 | 100 | 100 | 100 |
| | Wild Oats | 100 | 100 | 100 | 90 | 40 |
| | Yellow Foxtail | 100 | 100 | 100 | 100 | 90 |

## Claims

1. A compound having the formula

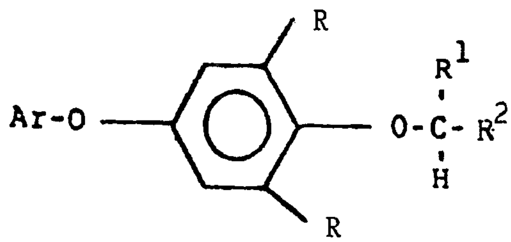

wherein Ar represents the group

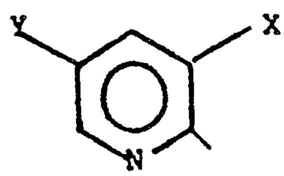

wherein X is halogen and Y is halogen, $CF_3$, $CHF_2$ or $CClF_2$;
R is H or F with the proviso that at least one R is F;
$R^1$ is a $C_1$-$C_3$ alkyl group;
$R^2$ is a carboxyl group; an agriculturally acceptable alkali metal, alkaline earth metal or ammonium salt thereof; or a $C_{1-6}$ alkyl ester thereof.

2. A compound as claimed in claim 1 wherein X is Cl and y is Cl.

3. A compound as claimed in claim 1 wherein X is F and Y is Cl.

4. A compound as claimed in claim 1 wherein x is Cl and y is $CF_3$.

5. A compound as claimed in any one of claims 1 to 4 wherein $R^1$ is a methyl group; and $R^2$ is the group COOR" wherein R" is hydrogen, methyl, ethyl, propyl, isopropyl, isobutyl or n-butyl.

6. A compound as claimed in claim 5 wherein R" is H.

7. A compound as claimed in claim 5 wherein R" is methyl.

8. A compound as claimed in claim 1 of the formula

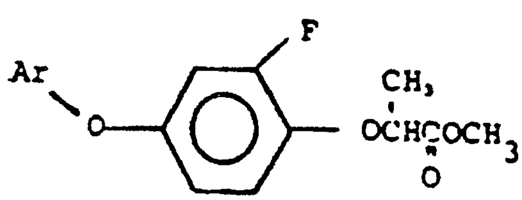

wherein the group Ar is

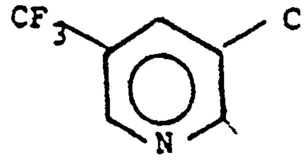 , 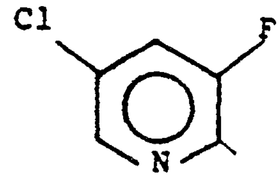 ,

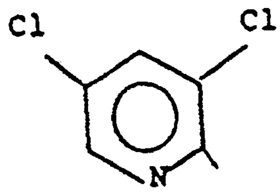 , or 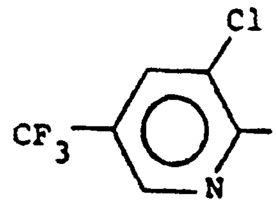

9. The R-enantiomer of a compound as claimed in any one of the preceding claims.

10. A herbicidal composition comprising an inert adjuvant or carrier and a herbicidally effective amount of a compound of claim 1 or an R-enantiomer thereof.

11. A composition as claimed in claim 10 wherein the said active compound is a compound as defined in any one of claims 2 to 9.

12. Process for making a compound having the formula

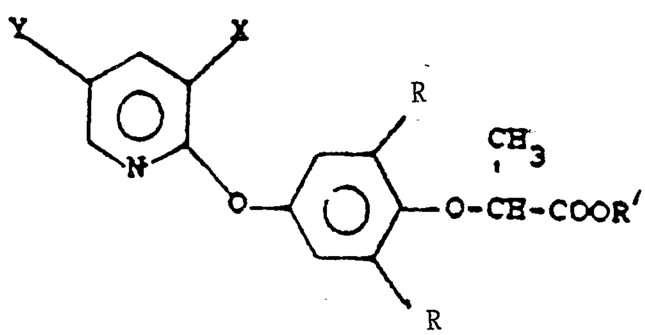

wherein R, X and Y are as defined in claim 1, and R' is hydrogen or methyl, which comprises reacting a compound having the formula

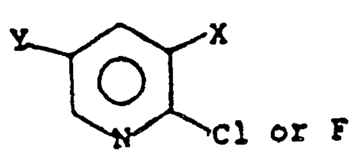

wherein X and Y as defined above with a compound having the formula

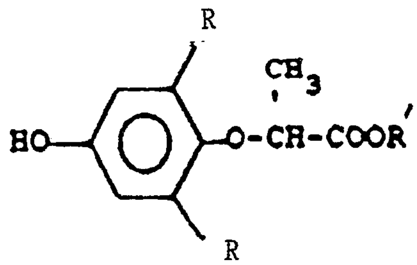

wherein R and R' are so defined above, in the presence of a base.

**13.** A method of controlling undesired plant growth which comprises applying to the locus of the plants a composition as claimed in claim 10 or claim 11.

**14.** A method as claimed in claim 13 wherein the composition is applied postemergently to grassy weeds.

**15.** A method as claimed in claim 14 wherein the composition is applied at a dosage rate sufficient to provide from 0.005 to 20 pounds/acre (0.0056 - 22.4kg/hectare) of the active ingredient.

**16.** A method as claimed in claim 13 wherein the composition is applied pre-emergently.

**17.** A method as claimed in claim 16 wherein the composition is applied pre-emergently at a dosage rate sufficient to provide from 0.05 to 2.0lbs/acre (0.056 to 2.25 kgs/hectare) of the active ingredient.

**18.** A method of killing or controlling vegetation, which method comprises providing in the vegetation a compound of claim 1.

Claims for the following Contracting State: AT

**1.** A herbicidal composition comprising an inert adjuvant or carrier and a herbicidally effective amount of a compound having the formula

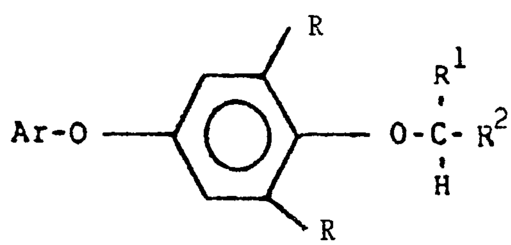

wherein Ar represents the group

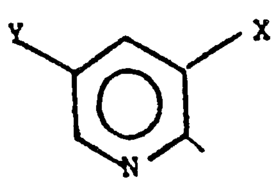

wherein X is halogen and Y is halogen, $CF_3$, $CHF_2$ or $CClF_2$;
R is H or F with the proviso that at least one K is F;
$R^1$ is a $C_1$-$C_3$ alkyl group;
$R^2$ is a carboxyl group; an agriculturally acceptable alkali metal, alkaline earth metal or ammonium salt thereof; or a $C_{1-6}$-

alkyl ester thereof.

2. A composition as claimed in claim 1 wherein the compound has the formula

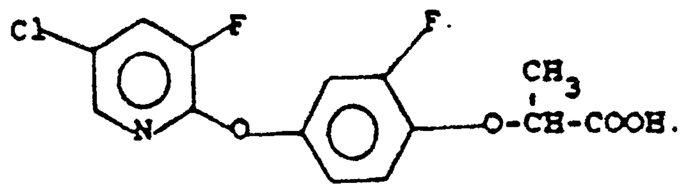

3. A composition as claimed in claim 1 wherein the compound has the formula

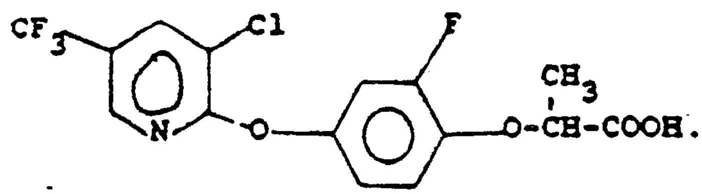

4. A composition as claimed in claim 1 wherein the compound has the formula

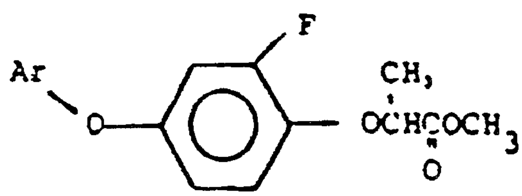

wherein the group Ar is

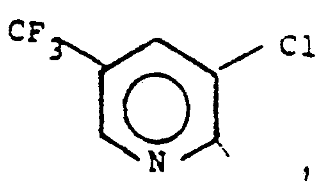 , 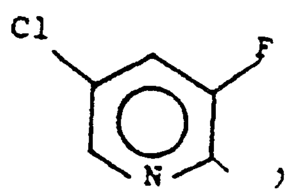 , 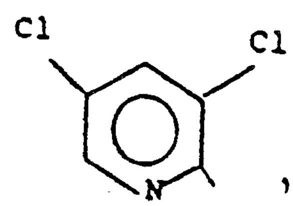 ,

or 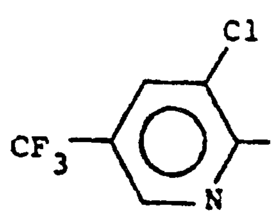

5. A process for the preparation of a compound of the formula

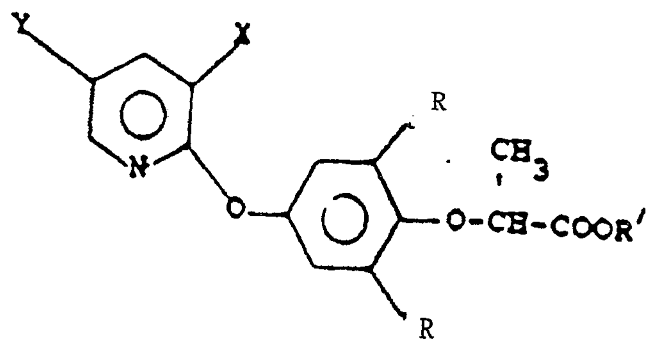

wherein R, X and Y are as defined in claim 1, and R' is hydrogen or methyl, which comprises reacting a compound having the formula

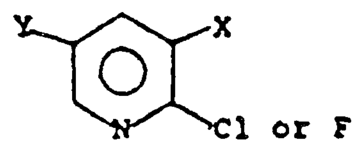

wherein X and Y are as defined above, with a compound having the formula

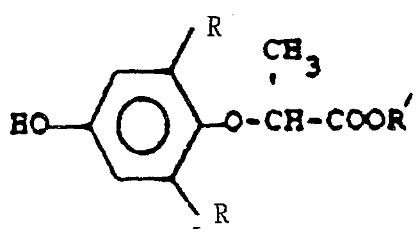

wherein R and R' are as defined above, in the presence of a base.

6. A method of controlling undesired plant growth which comprises applying to the locus of the plants a composition as claimed in any one of claims 1 to 4.

7. A method as claimed in claim 6 wherein the composition is applied postemergently to grassy weeds.

8. A method as claimed in claim 7 wherein the composition is applied at a dosage rate sufficient to provide from 0.005 to 20 pounds/acre (0.0056 - 22.4kg/hectare) of the active ingredient.

9. A method as claimed in claim 6 wherein the composition is applied preemergently.

10. A method as claimed in claim 9 wherein the composition is applied preemergently at a dosage rate sufficient to provide from 0.05 to 2.0lbs/acre (0.056 to 2.25kgs/hectare) of the active ingredient.

11. A method of killing or controlling vegetation, which method comprises providing in the vegetation a compound of claim 1.

**Revendications**

1. Composé de formule

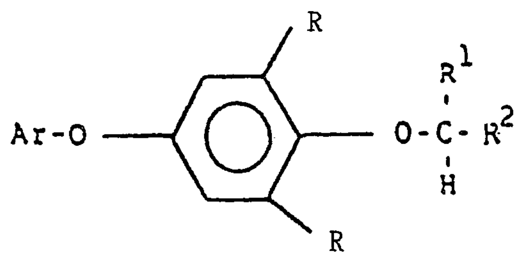

dans laquelle Ar représente le groupe

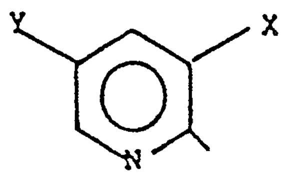

dans lequel X représente un atome d'halogène et Y représente un atome d'halogène ou un groupe $CF_3$, $CHF_2$ ou $CClF_2$ ;

R représente H ou F, avec la condition qu'au moins un R représente F ;

$R^1$ représente un groupe alkyle en $C_1$-$C_3$ ;

$R^2$ représente un groupe carboxyle ;

un sel de métal alcalin, de métal alcalino-terreux ou d'ammonium de ce composé, acceptable en agriculture ; ou un ester d'alkyle en $C_{1-6}$ de ce composé.

2. Composé tel que revendiqué dans la revendication 1, dans lequel X représente Cl et Y représente Cl.

3. Composé tel que revendiqué dans la revendication 1, dans lequel X représente F et Y représente Cl.

4. Composé tel que revendiqué dans la revendication 1, dans lequel X représente Cl et Y représente $CF_3$.

5. Composé tel que revendiqué dans l'une quelconque des revendications 1 à 4, dans lequel $R^1$ représente un groupe méthyle, et $R^2$ représente le groupe COOR", R" représentant un atome d'hydrogène ou un groupe méthyle, éthyle, propyle, isopropyle, isobutyle ou n-butyle.

6. Composé tel que revendiqué dans la revendication 5, dans lequel R" représente H.

7. Composé tel que revendiqué dans la revendication 5, dans lequel R" représente un groupe méthyle.

8. Composé tel que revendiqué dans la revendication 1, de formule

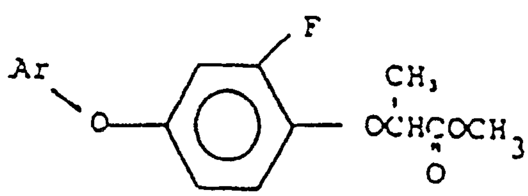

dans laquelle le groupe Ar représente

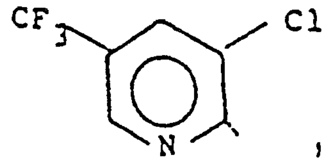 , 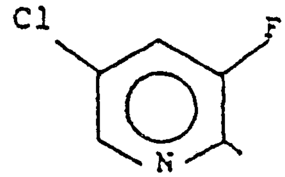 ,

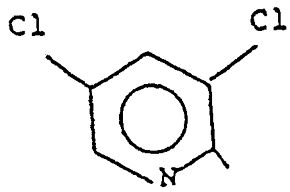 , ou 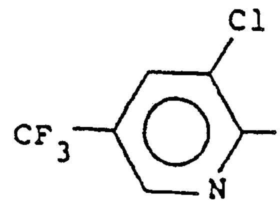

9. Enantiomère R d'un composé tel que revendiqué dans l'une quelconque des revendications précédentes.

10. Composition herbicide, comprenant un adjuvant ou un véhicule inerte, et une quantité efficace d'un composé herbicide selon la revendication 1 ou de son énantiomère R.

11. Composition telle que revendiquée dans la revendication 10, dans laquelle ledit composé actif est un composé tel que défini dans l'une quelconque des revendications 2 à 9.

12. Procédé de synthèse d'un composé présentant la formule

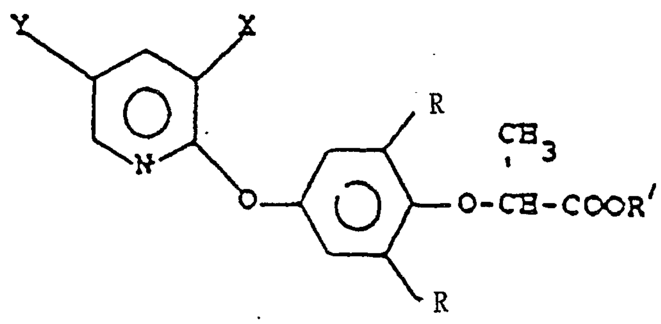

dans laquelle R, X et Y sont tels que définis dans la revendication 1 et R' représente un atome d'hydrogène ou un groupe méthyle, lequel procédé comporte la réaction d'un composé de formule

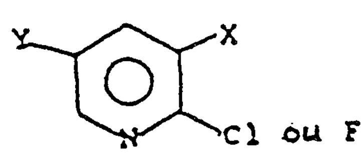

dans lequel X et Y sont tels que définis ci-dessus, avec un composé de formule

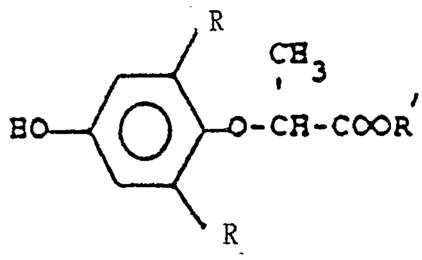

dans laquelle R et R' sont tels que définis ci-dessus, en présence d'une base.

**13.** Procédé de lutte contre la croissance indésirable de plantes, qui comprend 1'épandage, sur les lieux de pousse de ces plantes, d'une composition telle que revendiquée dans la revendication 10 ou la revendication 11.

**14.** Procédé tel que revendiqué dans la revendication 13, dans lequel on épand la composition sur des mauvaises herbes herbacées, après leur émergence.

**15.** Procédé tel que revendiqué dans la revendication 14, dans lequel la composition est épandue à une dose suffisante pour fournir de 0,005 à 20 livres par acre (0,0056 à 22,4 kg/ha) de l'ingrédient actif.

**16.** Procédé tel que revendiqué dans la revendication 13, dans lequel la composition est épandue avant l'émergence.

**17.** Procédé tel que revendiqué dans la revendication 16, dans lequel la composition est épandue, avant l'émergence, à une dose suffisante pour fournir de 0,05 à 2,0 livres par acre (0,056 à 2,25 kg/ha) de l'ingrédient actif.

**18.** Procédé pour détruire la végétation ou lutter contre elle, lequel procédé comprend l'épandage d'un composé de la revendication 1 sur la végétation.

Revendication pour l'Etat contractant suivant: AT

**1.** Composition herbicide comprenant un adjuvant ou un véhicule inerte et une quantité efficace d'un composé herbicide de formule

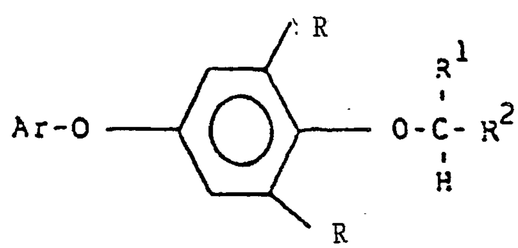

dans laquelle Ar représente le groupe

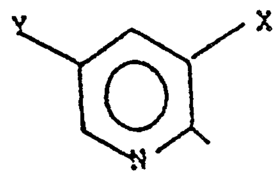

dans lequel X représente un atome d'halogène et Y représente un atome d'halogène ou un groupe $CF_3$, $CHF_2$ ou $CClF_2$ ;

R représente H ou F, avec la condition qu'au moins un R représente F ;

$R^1$ représente un groupe alkyle en $C_1$-$C_3$ ;
$R^2$ représente un groupe carboxyle ;
un sel de métal alcalin, de métal alcalino-terreux ou d'ammonium de ce composé, acceptable en agriculture ; ou un ester d'alkyle en $C_{1-6}$ de ce composé.

2. Composition telle que revendiquée dans la revendication 1, dans laquelle le composé présente la formule

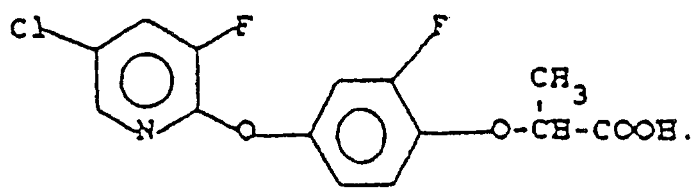

3. Composition telle que revendiquée dans la revendication 1, dans laquelle le composé présente la formule

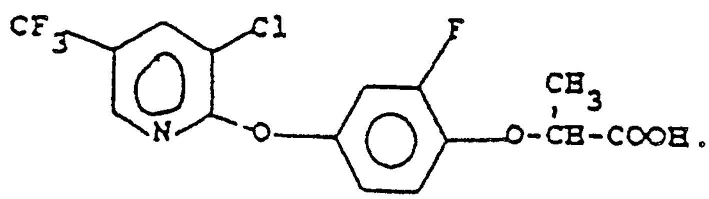

4. Composition telle que revendiquée dans la revendication 1, dans laquelle le composé présente la formule

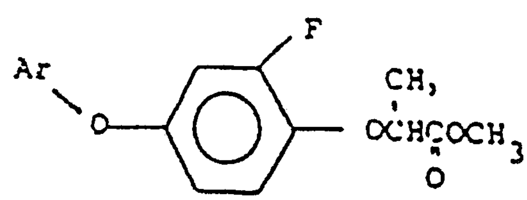

dans laquelle le groupe Ar représente

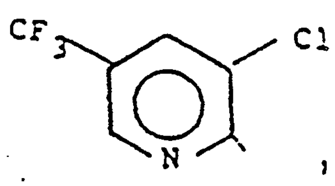 , 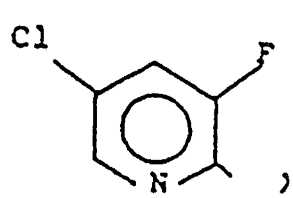 , 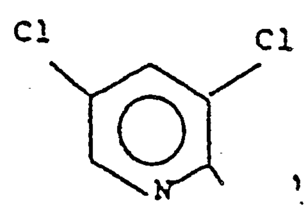

ou 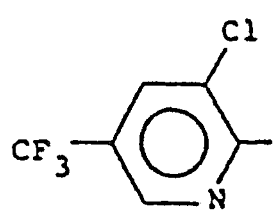

5. Procédé de préparation d'un composé de formule

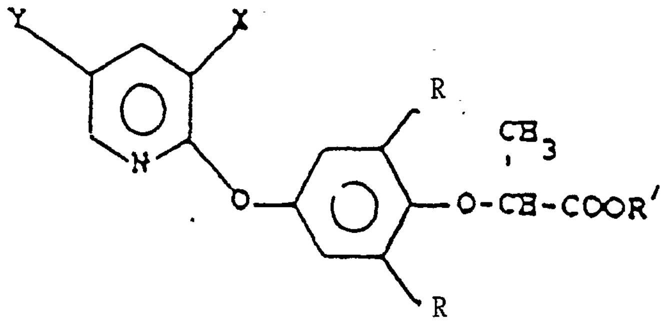

dans laquelle R, X et Y sont tels que définis dans la revendication 1 et R' représente un atome d'hydrogène ou un groupe méthyle, qui comprend la réaction d'un composé de formule

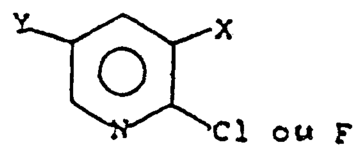

dans laquelle X et Y sont tels que définis ci-dessus, avec un composé de formule

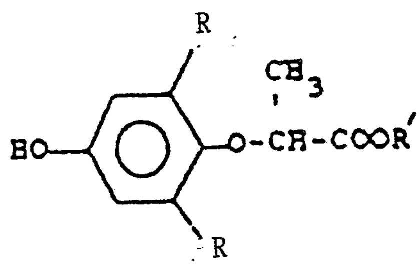

dans laquelle R et R' sont tels que définis ci-dessus, en présence d'une base.

6. Procédé de lutte contre la croissance indésirable de plantes, qui comprend l'épandage, sur le lieu de croissance des plantes, d'une composition telle que revendiquée dans l'une quelconque des revendications 1 à 4.

7. Procédé tel que revendiqué dans la revendication 6, dans lequel on épand la composition sur des mauvaises herbes herbacées, après leur émergence.

8. Procédé tel que revendiqué dans la revendication 7, dans lequel on épand la composition à une dose suffisante pour fournir de 0,005 à 20 livres/acre (0, 0056-22,4 kg/ha) de l'ingrédient actif.

9. Procédé tel que revendiqué dans la revendication 6, dans lequel on épand la composition après l'émergence.

10. Procédé tel que revendiqué dans la revendication 9, dans lequel on épand la composition, après l'émergence, à une dose suffisante pour fournir de 0,05 à 2,0 livres/acre (0,0056 à 2,25 kg/ha) de l'ingrédient actif.

11. Procédé pour détruire la végétation ou lutter contre elle, lequel procédé comprend l'épandage d'un composé de la revendication 1 sur la végétation.

## Ansprüche

1. Verbindung der Formel

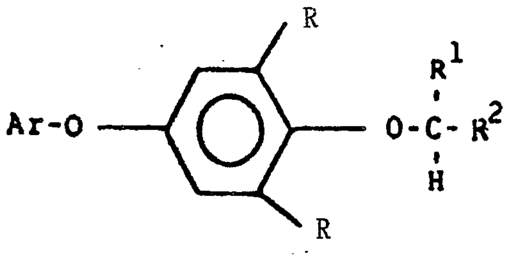

worin Ar die Gruppe bedeutet:

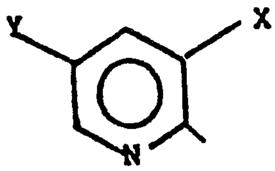

worin x = Halogen und Y = Halogen, $CF_3$, $CHF_2$ oder $CClF_2$ ist;
R = H oder F ist, mit der Maßgabe, daß wenigstens ein R = F ist;
$R^1$ eine $c_1$-$C_3$-Alkylgruppe ist;
$R^2$ eine Carboxylgruppe, ein landwirtschaftlich annehmbares Alkalimetall-, Erdalkalimetall- oder Ammoniumsalz hiervon oder ein $C_{1-6}$ Alkylester hiervon ist.

2. Verbindung nach Anspruch 1, worin x = Cl und Y = Cl ist.

3. Verbindung nach Anspruch 1, worin X = F und Y = Cl ist.

4. Verbindung nach Anspruch 1, worin X = Cl und Y = $CF_3$ ist.

5. Verbindung nach einem der Ansprüche 1 bis 4, worin $R^1$ eine Methylgruppe ist und $R^2$ die Gruppe COOR" ist, worin R" Wasserstoff, Methyl, Ethyl, Propyl, Isopropyl, Isobutyl oder n-Butyl ist.

6. Verbindung nach Anspruch 5, worin R" = H ist.

7. Verbindung nach Anspruch 5, worin R" Methyl ist.

8. Verbindung nach Anspruch 1 der Formel

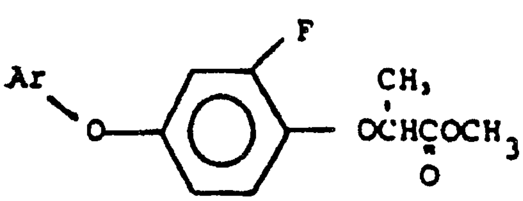

worin die Gruppe Ar ist:

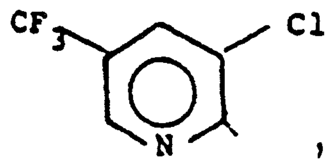,
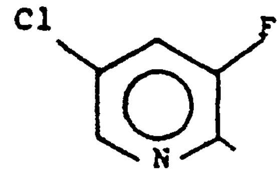,

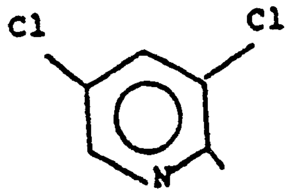, oder 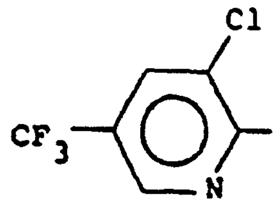

9. Das R-Enantiomere einer Verbindung nach einem der vorhergehenden Ansprüche.

10. Herbicidzusammensetzung, umfassend einen inerten Hilfsstoff oder Träger und eine herbicid-wirksame Menge einer Verbindung von Anspruch 1 oder ein R-Enantiomeres hiervon.

11. Zusammensetzung nach Anspruch 10, worin diese aktive Verbindung eine Verbindung nach der Definition in einem beliebigen der Ansprüche 2 bis 9 ist.

12. Verfahren zur Herstellung einer Verbindung der Formel:

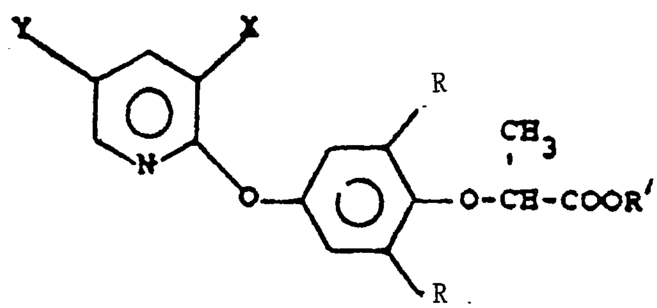

worin R, X und Y die in Anspruch 1 angegebene Bedeutung besitzen und R' Wasserstoff oder Methyl ist, wobei es umfaßt die Umsetzung einer Verbindung der Formel:

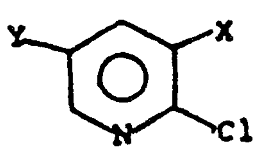 oder F

worin X und Y die zuvor angegebene Bedeutung besitzen, mit einer Verbindung der Formel:

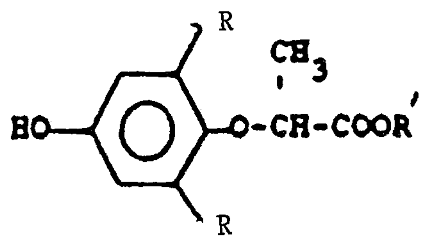

worin R und R' die zuvor angegebene Bedeutung besitzen, in Anwesenheit einer Base.

**13.** Verfahren zur Steuerung des unerwünschten Pflanzenwachstums, welches den Auftrag einer Zusammensetzung nach Anspruch 10 oder Anspruch 11 auf den Ort der Pflanzen umfaßt.

**14.** Verfahren nach Anspruch 13, bei welchem die Zusammensetzung nach dem Hervorkommen auf Grassaat aufgebracht wird.

**15.** Verfahren nach Anspruch 14, worin die Zusammensetzung mit einer ausreichenden Dosierungsmenge aufgebracht wird, um von 0,005 bis 20 Pounds/Acre (0,0056 - 22,4 kg/ Hektar) an aktivem Inhaltsstoff bereitzustellen.

**16.** Verfahren nach Anspruch 13, bei welchem die Zusammensetzung vor dem Hervorkommen aufgebracht wird.

**17.** Verfahren nach Anspruch 16, bei welchem die Zusammensetzung vor dem Hervorkommen mit einer ausreichenden Dosierungsmenge aufgebracht wird, um von 0,05 bis 2,0 lbs/Acre (0,056 bis 2,25 kg/Hektar) an aktivem Inhaltsstoff bereitzustellen.

**18.** Verfahren zum Abtöten oder zur Steuerung von Vegetation, wobei das Verfahren die Bereitstellung einer Verbindung von Anspruch 1 in der Vegetation umfaßt.

Patentansprüche für folgenden Vertragsstaat: AT

**1.** Herbicide Zusammensetzung, umfassend einen inerten Hilfsstoff oder Träger und eine herbicidwirksame Menge einer Verbindung der Formel:

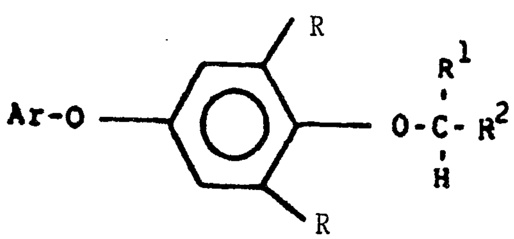

worin Ar die Gruppe bedeutet:

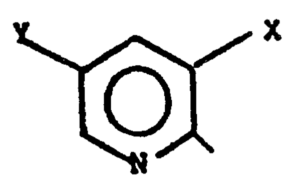

worin X = Halogen und Y = Halogen, $CF_3$, $CHF_2$ oder $CClF_2$ ist;
R = H oder F ist, mit der Maßgabe, daß wenigstens ein R = F ist;

$R^1$ eine $C_1$-$C_3$-Alkylgruppe ist;
$R^2$ eine Carboxylgruppe, ein landwirtschaftlich annehm bares Alkalimetall-, Erdalkalimetall- oder Ammonium salz hiervon oder ein $C_{1\text{-}6}$ Alkylester hiervon ist.

2. Zusammensetzung nach Anspruch 1, worin die Verbindung die Formel besitzt:

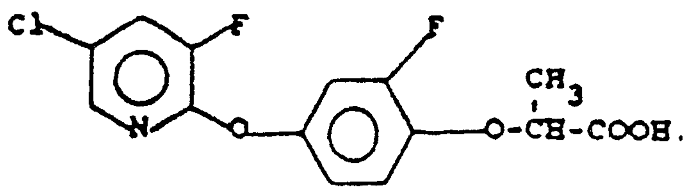

3. Zusammensetzung nach Anspruch 1, worin die Verbindung die Formel besitzt:

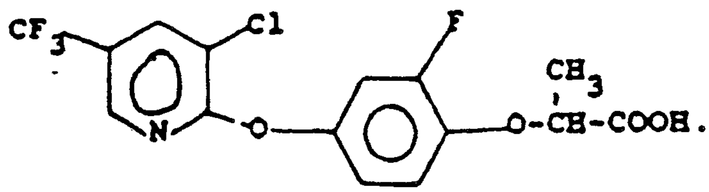

4. Zusammensetzung nach Anspruch 1, worin die Verbindung die Formel besitzt:

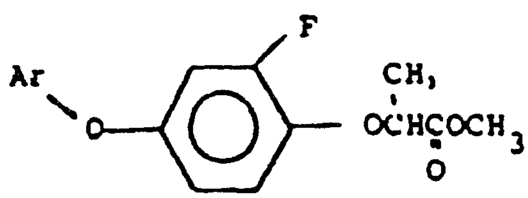

worin die Gruppe Ar ist:

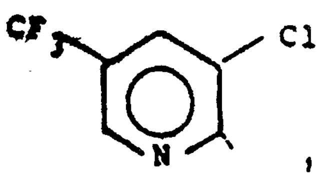 , 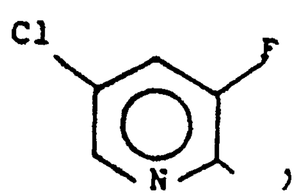 , 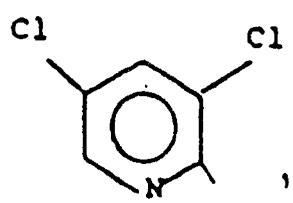 ,

oder 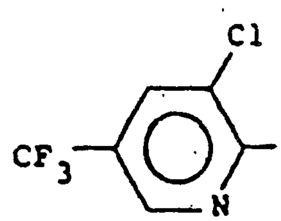

5. Verfahren zur Herstellung einer Verbindung der Formel:

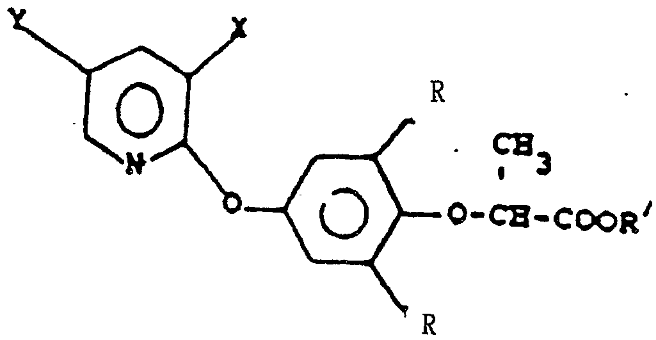

worin R, X und Y die in Anspruch 1 angegebene Bedeutung besitzen und R' Wasserstoff oder Methyl ist, wobei es umfaßt die Umsetzung einer Verbindung der Formel:

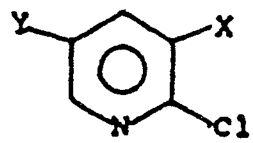 oder F

worin X und Y die zuvor angegebene Bedeutung besitzen, mit einer Verbindung der Formel:

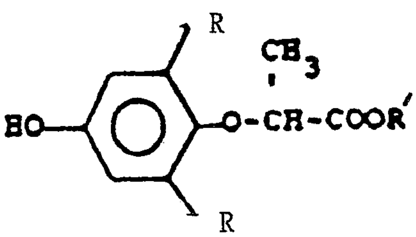

worin R und R' die zuvor angegebene Bedeutung besitzen, in Anwesenheit einer Base.

6. Verfahren zur Steuerung des unerwünschten Pflanzenwachstums, welches den Auftrag einer Zusammensetzung nach einem der Ansprüche 1 bis 4 auf den Ort der Pflanzen umfaßt.

7. Verfahren nach Anspruch 6, bei welchem die Zusammensetzung nach dem Hervorkommen auf Grassaat aufgebracht wird.

8. Verfahren nach Anspruch 7, worin die Zusammensetzung mit einer ausreichenden Dosierungsmenge aufgebracht wird, um von 0,005 bis 20 Pounds/Acre (0,0056 - 22,4 kg/ Hektar) an aktivem Inhaltsstoff bereitzustellen.

9. Verfahren nach Anspruch 6, bei welchem die Zusammensetzung vor dem Hervorkommen aufgebracht wird.

10. Verfahren nach Anspruch 9, bei welchem die Zusammensetzung vor dem Hervorkommen mit einer ausreichenden Dosierungsmenge aufgebracht wird, um von 0,05 bis 2,0 lbs/Acre (0,056 bis 2,25 kg/Hektar) an aktivem Inhaltsstoff bereitzustellen.

11. Verfahren zum Abtöten oder zur Steuerung von Vegetation, wobei das Verfahren die Bereitstellung einer Verbindung von Anspruch 1 in der Vegetation umfaßt.